# EUROPEAN PATENT APPLICATION

(11) **EP 4 516 229 A1**
(43) Date of publication of application: **05.03.2025**
(21) Application number: 23796584.3
(22) Date of filing: 06.02.2023
(51) Int. Cl.: A61B 6/00, A61B 6/14, A61B 6/03, A61B 5/00, G06T 7/30

(54) **APPARATUS AND METHOD FOR MATCHING DATA THROUGH SIMULTANEOUS CAPTURING OF CONE BEAM CT AND 3-DIMENSIONAL SCAN**

(30) Priority: 27.04.2022 KR 20220051843
(71) Applicant: HDX Will Corp., Seoul 03162 (KR)
(72) Inventor: JUNG, Hong, Seoul 03162 (KR); LEE, Sung Min, Bucheon-si Gyeonggi-do 14549 (KR)
(74) Representative: Murgitroyd & Company
(86) International application number: PCT/KR2023/001656
(87) International publication number: WO 2023/210934

(57) **Abstract**

The present invention provides a method of matching data, which is performed by an apparatus for matching data, the method including synchronizing acquisition time points of cone beam computed tomography (CBCT) data and 3D scan data, conforming coordinate systems of the CBCT data and the 3D scan data, constructing a 3D scan image in which a motion is corrected by matching depth maps created each time the 3D scan data are acquired, and constructing a CBCT 3D image in which the motion is corrected from the CBCT data by applying motion information acquired by matching the depth maps.

## Description

### [Technical Field]

The present invention relates to a data matching technology, and more particularly, to an apparatus and method for matching data, which are capable of automatically matching a cone beam CT image and a three-dimensional scan image.

### [Background Art]

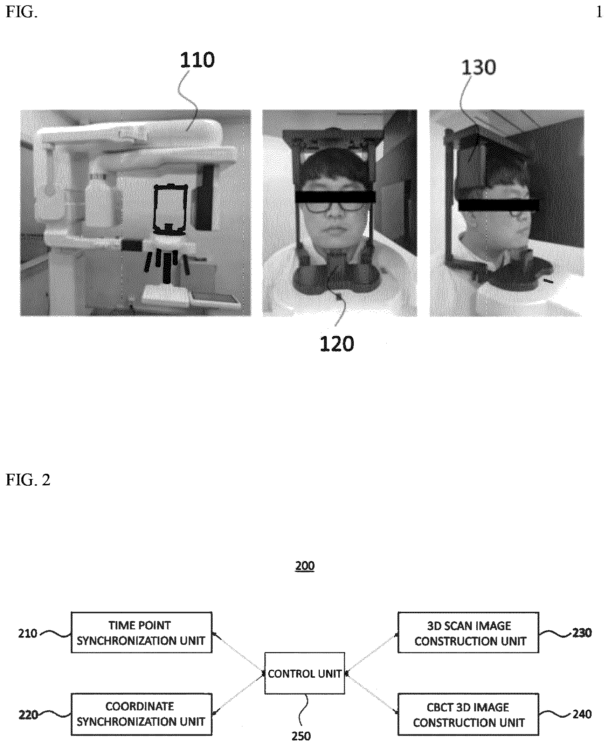

Recently, techniques have been developed to model and diagnose patients by additionally matching a 3D face scan image on a dental CBCT image. The reason why the 3D face scan image is additionally utilized is to virtually predict in advance a change in a patient's face and realistically show a current appearance often during orthodontic treatment or plastic surgery. However, with reference to FIG. 1, a dental CBCT device 110 in the related art fixes a patient's chin by placing the patient's chin on a chin rest 120 and fixes temple sites by using additional fixing mechanism 130 in order to minimize a motion of the patient's head during an imaging process. It is possible to forcibly prevent the motion of the patient during the process of capturing images of the patient by using fixing mechanisms such as the chin rest 120 and the additional fixing mechanisms 130. However, the chin is pressed by the chin rest 120, and the two lateral sides of the appearance of the patient, which are the temple sites, are covered by the additional fixing mechanisms 130 during the 3D face scan imaging process, which makes it impossible to represent an actual appearance of the patient at ordinary times. That is, even though the 3D face scan image is captured to realistically identify the current appearance of the patient, the fixing mechanism makes it difficult to identify the realistic appearance, which causes a problem in that it is difficult to utilize the 3D face scan image.

### [Document of Related Art]

### [Patent Document]

(Patent Document 1) Korean Patent No. 10-2273437

### [Disclosure]

### [Technical Problem]

An object of the present invention is to provide an apparatus and method for matching data, which are capable of capturing images of actual appearances at ordinary times by excluding a fixing mechanism, which is used to fix a patient's face, during a process of capturing a CBCT image and a 3D face scan image, and capable of obtaining a clear 3D image by correcting a motion of the patient even though the patient moves because of the absence of the fixing mechanism.

Technical problems to be solved by the present invention are not limited to the above-mentioned technical problems, and other technical problems, which are not mentioned above, may be clearly understood by those skilled in the art from the following descriptions.

### [Technical Solution]

In order to achieve the above-mentioned objects, a first aspect of the present invention provides a method of matching data, which is performed by an apparatus for matching data, the method including: synchronizing acquisition time points of cone beam computed tomography (CBCT) data and 3D scan data; conforming coordinate systems of the CBCT data and the 3D scan data; constructing a 3D scan image in which a motion is corrected by matching depth maps created each time the 3D scan data are acquired; and constructing a CBCT 3D image in which the motion is corrected from the CBCT data by applying motion information acquired by matching the depth maps.

In particular, the synchronizing of the acquisition time points may include: conforming a frame rate of a CBCT imaging apparatus for acquiring the CBCT data and a frame rate of a 3D scan imaging apparatus for acquiring the 3D scan data; and conforming a time point at which the CBCT imaging apparatus begins to detect the CBCT data and a time point at which the 3D scan imaging apparatus begins to capture the 3D scan data.

In particular, the conforming of the coordinate systems may include: calculating a transform by using an iterative closest point (ICP) algorithm to minimize a matching error of the CBCT data and the 3D scan data; applying the transform to the 3D scan data; and moving the 3D scan data, to which the transform is applied, to the CBCT data.

In particular, the constructing of the 3D scan image may include: creating the depth map for each frame for which the 3D scan data are acquired; matching the two depth maps with a difference by using an ICP algorithm when it is identified that there is the difference between the created depth maps; and setting a transform, which is calculated by the ICP algorithm, to motion information to match the two depth maps.

In particular, the acquiring of the depth map may include: detecting a corresponding point between cameras or between the camera and a projector from a particular pattern when the camera of a 3D scan imaging apparatus captures a particular pattern outputted to an image capturing target from the projector of the 3D scan imaging apparatus for acquiring the 3D scan data; acquiring depth information for each pixel of the camera with respect to the corresponding point; and creating the depth map on the basis of the depth information.

In particular, the constructing of the CBCT 3D image may include: searching for CBCT data acquired at the acquisition time point of the 3D scan data to which the motion information is set; and performing back-projection by applying a transform, which is the motion information, to the searched CBCT data.

In order to achieve the above-mentioned objects, a second aspect of the present invention provides an apparatus for matching data, the apparatus including: a time point synchronization unit configured to synchronize acquisition time points of cone beam computed tomography (CBCT) data and 3D scan data; a coordinate synchronization unit configured to conform coordinate systems of the CBCT data and the 3D scan data; a 3D scan image construction unit configured to construct a 3D scan image in which a motion is corrected by matching depth maps created each time the 3D scan data are acquired; and a CBCT 3D image construction unit configured to construct a CBCT 3D image in which the motion is corrected from the CBCT data by applying motion information acquired by matching the depth maps.

In order to achieve the above-mentioned objects, a third aspect of the present invention provides a computer program stored in a computer-readable medium and configured to be executed to perform the method of matching data when an instruction of the computer program is executed.

### [Advantageous Effects]

According to the present invention described above, a fixing mechanism for fixing the patient's face to the imaging apparatus is not required, the CBCT image and the 3D face scan image, in which a motion blur caused by the patient's motion is corrected, may be simultaneously obtained, and additional data matching of the CBCT image and the 3D face scan image is not required after the imaging process.

### [Description of Drawings]

FIG. 1 is an exemplified view illustrating an imaging apparatus in the related art.
FIG. 2 is a block diagram illustrating an apparatus for matching data according to the exemplary embodiment of the present invention.
FIG. 3 is a flowchart illustrating a method of matching data according to the embodiment.
FIG. 4 is an exemplified view for explaining a process of conforming a coordinate system according to the embodiment.
FIG. 5 is an exemplified view for explaining a process of creating a depth map according to the embodiment.
FIG. 6 is an exemplified view for explaining a process of matching a depth map according to the embodiment.
FIG. 7 is an exemplified view for explaining a process of constructing a CBCT 3D image according to the embodiment.
FIG. 8 is an exemplified view illustrating a CBCT 3D image and a 3D scan image according to the embodiment.
FIG. 9 is an exemplified view illustrating an imaging apparatus according to the embodiment.

### [Modes of the Invention]

Hereinafter, advantages and features of the present invention and methods of achieving the advantages and features will be clear with reference to embodiments described in detail below together with the accompanying drawings. However, the present invention is not limited to the embodiments disclosed herein but will be implemented in various forms. The embodiments of the present invention are provided so that the present invention is completely disclosed, and a person with ordinary skill in the art to which the present invention pertains can fully understand the scope of the present invention. The present invention will be defined only by the scope of the appended claims. Throughout the specification, the same reference numerals denote the same constituent elements. The term "and/or" includes each and all combinations of one or more of the mentioned items.

Terms "first", "second", and the like may be used to describe various elements, components, and/or sections, but the elements, components, and/or sections are of course not limited by these terms. These terms are merely used to distinguish one element, component, or section from other elements, components, or sections. Therefore, the first element, the first constituent element, or the first section mentioned hereinafter may of course be the second element, the second constituent element, or the second section within the technical spirit of the present invention.

In addition, the reference numerals (e.g., 'a,' 'b,' and 'c') used in operations are used for descriptive convenience and are not intended to describe the order of operations and the operations may be performed in a different order unless otherwise stated. That is, each of the steps may be performed in the same order as specified, may be performed substantially simultaneously, or may be performed in the reverse order.

The terms used in the present specification are for explaining the exemplary embodiments, not for limiting the present invention. Unless particularly stated otherwise in the present specification, a singular form also includes a plural form. The terms "comprise (include)" and/or "comprising (including)" used in the specification are intended to specify the presence of the mentioned constituent elements, steps, operations, and/or elements, but do not exclude presence or addition of one or more other constituent elements, steps, operations, and/or elements.

Unless otherwise defined, all terms (including technical and scientific terms) used in the present specification may be used as the meaning which may be commonly understood by the person with ordinary skill in the art, to which the present invention belongs. In addition, terms defined in a generally used dictionary shall not be construed in ideal or excessively formal meanings unless they are clearly and specially defined in the present specification.

In addition, in the description of the exemplary embodiments of the present invention, the specific descriptions of publicly known functions or configurations will be omitted when it is determined that the specific descriptions may unnecessarily obscure the subject matter of the present invention. In addition, the terms used in the exemplary embodiments of the present invention are defined considering the functions in the present invention and may vary depending on the intention or usual practice of a user or an operator. Therefore, the definition of the disclosure should be made based on the entire contents of the present specification.

FIG. 2 is a block diagram illustrating an apparatus for matching data according to the exemplary embodiment of the present invention.

With reference to FIG. 2, an apparatus 200 for matching data includes a time point synchronization unit 210, a coordinate synchronization unit 220, a 3D scan image construction unit 230, a CBCT 3D image construction unit 240, and a control unit 250. In this case, the control unit 250 controls operations and flows of data of the time point synchronization unit 210, the coordinate synchronization unit 220, the 3D scan image construction unit 230, and the CBCT 3D image construction unit 240.

First, the apparatus 200 for matching data is an apparatus configured to construct a CBCT 3D image and a 3D scan image on the basis of cone beam computed tomography (cone beam CT. CBCT) data and three-dimensional 3D face scan data. In particular, the apparatus 200 for matching data may be provided separately from a CBCT imaging apparatus for acquiring CBCT data and a 3D scan imaging apparatus for acquiring 3D scan data, or the apparatus 200 for matching data may be integrated with the CBCT imaging apparatus or the 3D scan imaging apparatus. In case that the apparatus 200 for matching data is provided separately from the CBCT imaging apparatus or the 3D scan imaging apparatus, the apparatus 200 for matching data may be connected to the CBCT imaging apparatus or the 3D scan imaging apparatus, which is physically separated from the apparatus 200 for matching data, by means of a wireless network or in a wired manner, acquire the CBCT data or the 3D scan data, and perform a method of matching data to be described below. That is, the shape of the apparatus 200 for matching data according to the present invention is not limited as long as the apparatus 200 for matching data may acquire the CBCT data and the 3D scan data. The shape of the apparatus 200 for matching data may be easily modified by those skilled in the art to which the present invention pertains. In addition, because the CBCT imaging apparatus, the 3D scan apparatus, the CBCT data acquisition method, and the 3D scan data acquisition method are technologies widely known to the technical field to which the present invention pertains, detailed descriptions thereof will be omitted.

In particular, the apparatus 200 for matching data may be a computer configured to execute an application or program installed to perform the method of matching data. The apparatus 200 for matching data may have a user interface and control a process of inputting or outputting data. In this case, the computer may mean all types of hardware devices each including at least one processor. The computer may be understood as a concept also including a software configuration operated by a hardware device according to the embodiment. For example, the computer may be understood as a concept including all smartphones, tablet PCs, desktops, notebook computers, user clients, and applications operated by the respective devices. However, the present invention is not limited thereto.

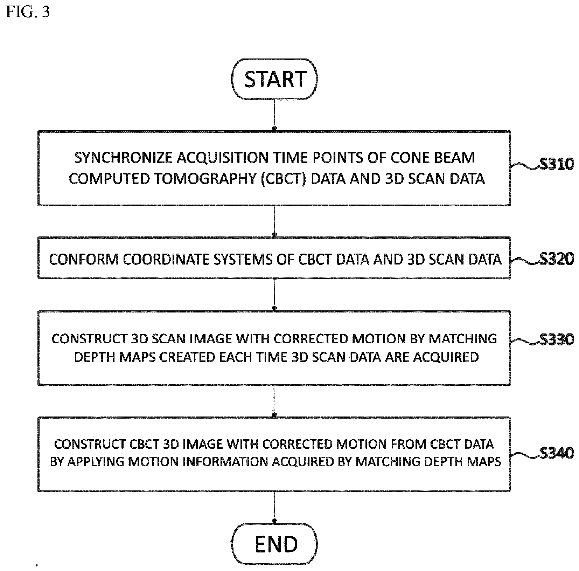

An operation performed by the components of the apparatus 200 for matching data illustrated in FIG. 2 will be described in detail with reference to FIG. 3. Steps, which will be described below with reference to FIG. 3, will be described as being performed by different components. However, the present invention is not limited thereto. At least some of the steps according to the embodiment may also be performed by identical or different components.

FIG. 3 is a flowchart illustrating the method of matching data according to the embodiment.

With reference to FIG. 3, the time point synchronization unit 210 synchronizes time points at which the CBCT data and the 3D scan data are acquired (step S310). In particular, the time point synchronization unit 210 may conform a frame rate of the CBCT imaging apparatus for acquiring the CBCT data and a frame rate of the 3D scan imaging apparatus for acquiring the 3D scan data. The time point synchronization unit 210 may conform a time point at which the CBCT imaging apparatus begins to detect the CBCT data and a time point at which the 3D scan imaging apparatus begins to capture the 3D scan data. That is, a detector of the CBCT imaging apparatus and a camera of the 3D scan imaging apparatus may be synchronized to identify the number of times the measurement of the 3D scan capturing is performed while corresponding to the time point at which the detector acquires data for each angle while a rotator of the CBCT imaging apparatus rotates.

For example, in case that the frame rate of the CBCT imaging apparatus corresponds to 30 FPS (frame per second) and the CBCT imaging apparatus acquires the CBCT data (i.e., projection data) corresponding to one frame per 1/30 seconds, the time point synchronization unit 210 may also set the frame rate of the 3D scan imaging apparatus to 30 FPS, and a start point at which the detector of the CBCT imaging apparatus acquires the CBCT data and a start point at which the 3D scan imaging apparatus captures the 3D scan data may be coincident with each other.

The coordinate synchronization unit 220 may conform coordinate systems of the CBCT data and the 3D scan data (step S320). In this case, the process in which the coordinate synchronization unit 220 conforms the coordinate systems may be provided to conform the coordinate systems by matching the CBCT data and the 3D scan data in a three-dimensional space and performed only once by using the CBCT data and the 3D scan data obtained by simultaneously or separately capturing CBCT images and 3D scan images of a stationary object that does not move. That is, the process may be performed before the imaging process of acquiring the CBCT 3D image and the 3D scan image of the patient is performed without the necessity of using data captured for the patient. However, the order in which the processes are performed is not limited thereto.

In particular, the coordinate synchronization unit 220 may calculate a transform by using an iterative closest point (ICP) algorithm to minimize a matching error of the CBCT data and the 3D scan data. Because the method of calculating the transform by using the ICP algorithm and the ICP algorithm may be easily performed by those skilled in the art to which the present invention pertains, a detailed description thereof will be omitted. The coordinate synchronization unit 220 may conform the coordinate systems of the two data by applying the calculated transform to the 3D scan data and moving the 3D scan data, to which the transform is applied, to the CBCT data.

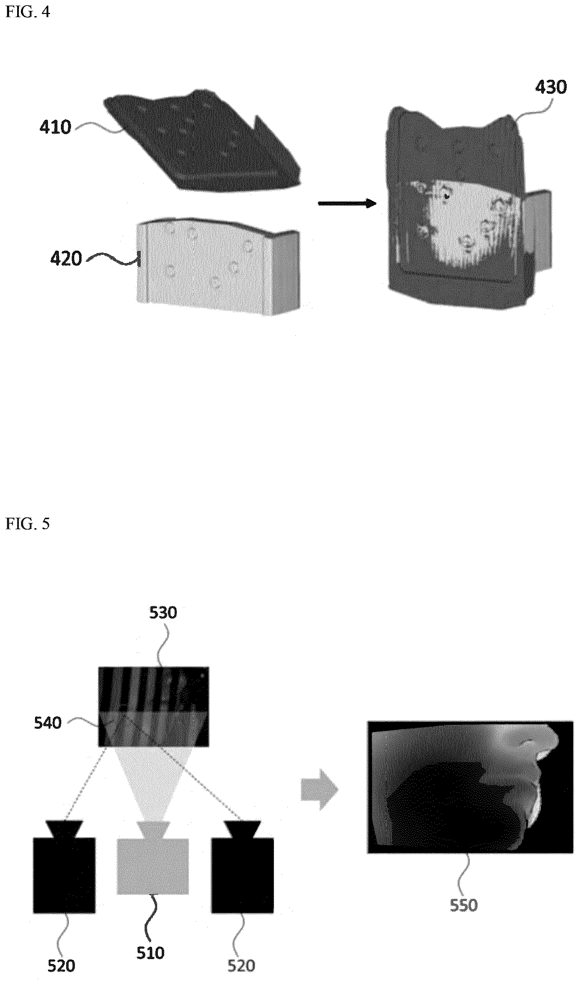

For example, with reference to FIG. 4 that is an exemplified view for explaining the process of conforming the coordinate systems, the coordinate synchronization unit 220 may acquire 3D scan data 410 and CBCT data 420 and calculate a transform T, in which ∥ TS-D∥ is minimized, by using the ICP algorithm when S represents the 3D scan data 410 and D represents the CBCT data 420. Next, with reference to the drawing indicated by reference numeral 430, the coordinates of the two data may be conformed by applying the calculated transform T to the 3D scan data 410 and moving the 3D scan data 410 to the CBCT data 420.

When the synchronization setting is completed through steps S310 and S320, the data matching is performed in real time together with the simultaneous capturing of the CBCT and the 3D scan without additional data matching after the CBCT data and the 3D scan data are captured, such that the CBCT 3D image and the 3D scan image may be immediately constructed. That is, unless the frame rate is changed, steps S310 and S320 may be performed only once, and then the CBCT 3D image and the 3D scan image may be acquired for the patient by repeatedly performing steps S330 and S340 each time the image of the patient is captured.

The 3D scan image construction unit 230 constructs the 3D scan image in which a motion is corrected by matching the created depth map each time the 3D scan data are acquired (step S330).

In particular, the 3D scan image construction unit 230 may create the depth map for each frame for which the 3D scan data are acquired. The depth map refers to a single image in three-dimensional computer graphics that contains information on a distance from an observation time point to a surface of the object. The process of creating the depth map will be described more specifically with reference to FIG. 5 that is an exemplified view for explaining the process of creating the depth map. The 3D scan imaging apparatus may include one or more projectors 510 and one or more cameras 520. When the projector 510 outputs a particular pattern to an image capturing target, the camera 520 may capture an image of the particular pattern outputted onto the image capturing target, and the 3D scan image construction unit 230 may detect a corresponding point between the camera and the particular pattern captured by the camera or between the camera and the projector. In this case, in accordance with the number of cameras or the number of proj ectors, the corresponding point may be found between the cameras or between the camera and the projector. Next, the 3D scan image construction unit 230 may acquire depth information for each pixel of the camera with respect to the detected corresponding point and create the depth map on the basis of the depth information. The depth map may be created for each frame. For example, when the frame rate is 30 FPS, the depth maps may be created for thirty frames per second.

In particular, when it is identified that there is a difference between the depth maps created for each frame, the 3D scan image construction unit 230 may match the two depth maps with a difference by using the ICP algorithm. When the patient, which is the image capturing target, moves during the imaging process, a difference occurs between the depth maps acquired from the 3D scan data. Therefore, it is possible to acquire the clear 3D scan image by matching the depth maps acquired for each frame, and the patient's motion occurring for each frame may be identified.

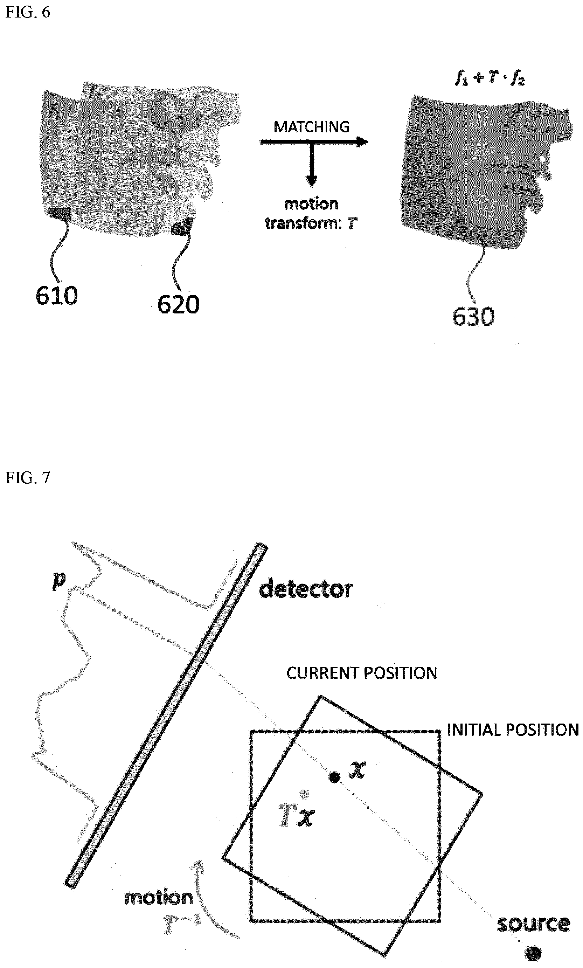

For example, with reference to FIG. 6 that is an exemplified view for explaining the process of matching the depth maps, a depth map created for a first frame is referred to as f1610, and a depth map created for a second frame is referred to as f2620. That is, when the depth map moves from f1610 to f2620 as the patient's motion occurs during the imaging process, the 3D scan image construction unit 230 may obtain the transform T for matching f2620 with f1610 by using the ICP algorithm. In this case, when T⁻¹, which is an inverse of the transform, corresponds to an actual motion of the patient and the transform T is applied to f2620, f1610 and f2620 are matched as illustrated in the drawing indicated by reference numeral 630.

In the embodiment, the depth maps created for each frame may be matched based on the first frame. That is, as shown in Table 1 below, based on a depth map f1 for the first frame, the 3D scan image construction unit 230 may match the depth maps created for each frame by obtaining the transform T2 for matching a depth map f2 for a second frame with f1 and applying T2 to f2 and by obtaining the transform T3 for matching a depth map f3 for a third frame with f1 and applying T3 to f3. The matching between the depth maps may be performed on all the frames that are criteria. Alternatively, the matching may not be performed on the frame having the depth map identical to the depth map that is a criterion.

**[Table 1]**

| Frame (Depth Map) | Transform | Matching |
|---|---|---|
| f1 | - | - |
| f2 | T2 | f1+T2·f2 |
| f3 | T3 | f1+T3·f3 |
| ... | ... | ... |
| f30 | T30 | f1+T30·f30 |

In another embodiment, the depth maps created for each frame may be matched based on the frame that satisfies a particular criterion. In this case, the particular criterion may correspond to a case in which the frame is a frame in a maximum section in which no difference occurs between the depth maps or a case in which a size of a section in which no difference occurs between the depth maps exceeds a preset section. That is, when the depth maps for all the frames are created, the 3D scan image construction unit 230 may identify whether a difference is present between the depth maps. In case that there is a section in which no difference is present, the matching may be performed by setting the depth map, which is related to the frame of the largest section among the corresponding sections, to the criterion of the matching. In case that the section in which no difference is present satisfies a section with a preset size, the matching may be performed by setting the frame, which belongs to the corresponding section, to the criterion of the matching of the depth maps.

For example, in case that the depth maps of the frames f2 to f4 are identical to one another and the depth maps of the frames f6 to f20 are identical to one another among the frames between the first frame f1 and the final frame fn, the section from f6 to f20 is the largest section in which no difference is present between the depth maps, such that the matching may be performed based on the frame corresponding to one of the frames f6 to f20 (here, based on f6). Alternatively, in case that the depth maps of the frames f6 to f18 are identical to one another and a size of a preset section is 10, the frames f6 to f18 satisfy the size of the section, such that the matching may be performed based on the frame corresponding to one of the frames f6 to f18 (here, based on f6). With reference to Table 2 below, based on a depth map f6 for a sixth frame, the 3D scan image construction unit 230 may match the depth maps created for each frame by obtaining the transform T1 for matching the depth map f1 for the first frame with f6 and applying T1 to f1 and by obtaining the transform T2 for matching the depth map f2 for the second frame with f6 and applying T2 to f2. Because the depth maps of f6 to f18 are identical to one another, the matching may not be performed.

**[Table 2]**

| Frame (Depth Map) | Transform | Matching |
|---|---|---|
| f1 | T1 | f6+T1·f1 |
| f2 | T2 | f6+T2·f2 |
| ... | ... | ... |
| f6 to f18 | - | - |
| ... | ... | ... |
| f29 | T29 | f6+T29·f29 |
| fn | Tn | f6+Tn·fn |

In particular, the 3D scan image construction unit 230 may set the transform, which is calculated on the basis of the ICP algorithm, to motion information to match the two depth maps. The 3D scan image construction unit 230 may be set by matching motion information for each frame. For example, in Table 1, the motion information of the frame f2 may be set to T2, the motion information of the frame f3 may be set to T3, and the motion information of the frame f30 may be set to T30.

As described above, the 3D scan image construction unit 230 simultaneously constructs the 3D scan image and acquires the motion information by matching the depth maps.

The CBCT 3D image construction unit 240 constructs the CBCT 3D image, in which the motion is corrected from the CBCT data, by applying the motion information acquired by matching the depth maps in step S330 (step S340). The CBCT data are constructed as three-dimensional images by back-projection, and an image with no motion blur is acquired by applying motion information acquired in step S330 when the CBCT data for each frame are back-projected.

In particular, the CBCT 3D image construction unit 240 may search for the CBCT data acquired at an acquisition time point of the 3D scan data to which the motion information is set. More specifically, the CBCT 3D image construction unit 240 identifies the frame of the 3D scan data to which the motion information is set on the basis of the motion information for each frame set in step S330 and identifies the CBCT data acquired at the time point at which the frame of the corresponding 3D scan data are acquired. Because the acquisition time point of the CBCT data and the acquisition time point of the 3D scan data are in a state of being synchronized in step S310, the CBCT 3D image construction unit 240 may find the CBCT data at the corresponding time point on the basis of what frame of the 3D scan data in which the motion occurs. In this case, the found CBCT data may correspond to the frame acquired at the time point, which is identical to the corresponding frame of the 3D scan data or the closest time point.

Next, the CBCT 3D image construction unit 240 may perform the back-projection by applying the transform, which is the motion information, to the searched CBCT data. With reference to FIG. 7 that is an exemplified view for explaining the process of constructing the CBCT 3D image, the CBCT 3D image construction unit 240 may restore the CBCT 3D image, in which the motion is corrected by allowing a back-projection value to correspond to Tx instead of x by using the transform T that is the motion information when the CBCT data in respect to an x-position are back-projected. That is, the back-projection is performed by applying motion information T obtained in step S330 when the CBCT data are constructed as 3D data.

For example, as shown in Table 1, in case that the 3D scan image construction unit 230 obtains the transform for each frame of the 3D scan data and sets the transform to the motion information. Therefore, the CBCT 3D image construction unit 240 may back-project CBCT data F1, which correspond to the first frame, in an intact manner because there is no motion information in respect to f1, back-project CBCT data F2, which correspond to the second frame, by applying T2 because the motion information in respect to f2 is T2, and back-project CBCT data F3, which correspond to the third frame, by applying T3 because the motion information in respect to f3 is T3. The CBCT 3D image construction unit 240 may construct the CBCT 3D image by performing the back-projection to the CBCT data corresponding to the final frame in the same way.

In this case, because the coordinate systems of the CBCT data and the 3D scan data have been conformed in step S320, the motion information acquired from the 3D scan data may be used in an intact manner when the 3D image of the CBCT data is constructed.

As described above, the CBCT 3D image construction unit 240 acquires the CBCT 3D image from the CBCT data by means of the back-projection to which the motion information is applied.

In addition, step S340 has been described as being performed after step S330, but steps S340 and S330 may be simultaneously performed in parallel. That is, because step S330 uses the 3D scan data and step S340 uses the CBCT data, the CBCT 3D image, to which the corresponding motion information is applied, may be simultaneously constructed in real time by means of step S340 each time the motion information is set for each frame by means of step S330.

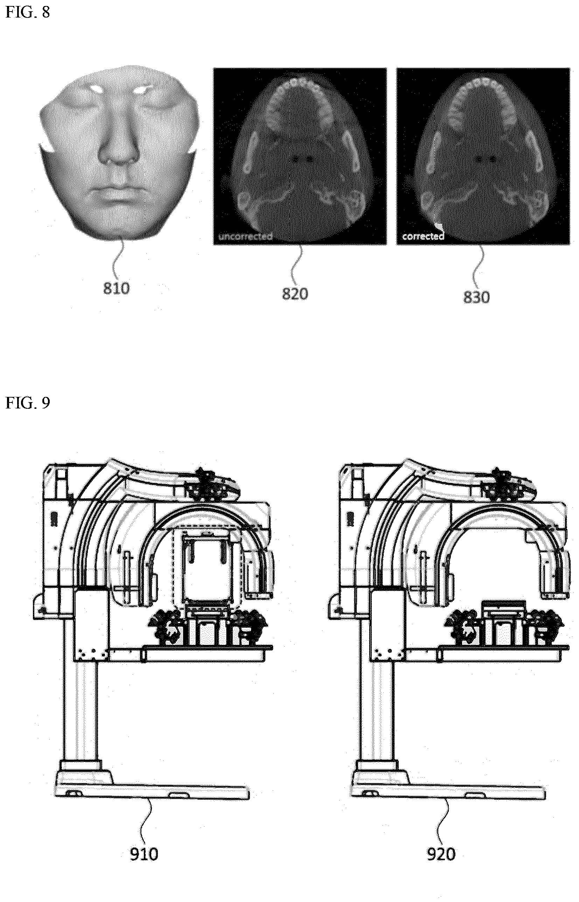

FIG. 8 is an exemplified view illustrating the CBCT 3D image and the 3D scan image according to the embodiment.

FIG. 8 illustrates a 3D scan image 810 in which a motion is corrected, a CBCT 3D image 820 in which a motion is not corrected, and a CBCT 3D image 830 in which a motion is corrected.

In particular, the 3D scan image 810, in which the motion is corrected, and the CBCT 3D image 830, in which the motion is corrected, are images acquired by the method of matching data according to the present invention. After steps S310 and S320 described with reference to FIG. 3 are performed, the 3D scan image 810 is acquired when the depth maps are matched by means of step S330 in respect to all the 3D scan data acquired for a particular patient, and the CBCT 3D image 830 is acquired when the back-projection is performed by means of step S340 in respect to the CBCT data for all angles.

When the CBCT 3D image 820, in which the motion is not corrected, and the CBCT 3D image 830, in which the motion is corrected, are compared, it can be seen that the CBCT 3D image 820, in which the motion is not corrected, has a motion blur because of the patient's motion, and the CBCT 3D image 830, in which the motion is corrected, is acquired as a clear image because the motion is corrected by applying the transform calculated during the process of matching the depth maps of the 3D scan image.

FIG. 9 is an exemplified view illustrating an imaging apparatus according to the embodiment.

As indicated by a red dotted line indicating an imaging apparatus corresponding to reference numeral 910, a fixing mechanism, which fixes the patient's face and prevents a motion of the patient's face, is essentially provided to capture the CBCT image in the related art. In contrast, according to the present invention, the CBCT 3D image and the 3D scan image, in which the motions are corrected, may be immediately acquired even though the patient during the imaging process, such that the structure of the imaging apparatus may be designed without a fixing mechanism, like the imaging apparatus corresponding to reference numeral 920. The structure of the imaging apparatus illustrated in FIG. 9 is provided to explain the presence or absence of the fixing mechanism. However, the present invention is not limited to the corresponding structure, and the structure of the imaging apparatus may be modified to any structure as long as the structure does not have the fixing mechanism.

Meanwhile, the steps of the described method or algorithm related to the embodiment of the present invention may be directly implemented by a hardware module, implemented by a software module executed by hardware, or implemented by a combination thereof. The software module may reside in a random access memory (RAM), a read-only memory (ROM), an erasable programmable ROM (EPROM), an electrically erasable programmable ROM (EEPROM), a flash memory, a hard disc, a removable disc, a CD-ROM, or a computer-readable recording medium in any form well known to the technical field to which the present invention pertains.

The constituent elements of the present invention may be implemented as a program (or application) and stored in a medium so as to be executed by being combined with a computer that is hardware. The constituent elements of the present invention may be executed as software programs or software elements. Similarly, the embodiments may be implemented in programming or scripting languages, such as C, C++, Java, assembler, and the like, including various algorithms implemented as data structures, processes, routines, or combinations of other programming configurations. The functional aspects may be implemented as algorithms executed by one or more processors.

While the exemplary embodiments of the apparatus and method for matching data according to the present invention have been described above, the present invention is not limited thereto, and various modifications can be made and carried out within the scope of the claims, the detailed description of the invention, and the accompanying drawings, and also fall within the scope of the present invention.

### [Description of Reference Numerals]

- 200:: Apparatus for matching data
- 210:: Time point synchronization unit
- 220:: Coordinate synchronization unit
- 230:: 3D scan image construction unit
- 240:: CBCT 3D image construction unit
- 250:: Control unit

## Claims

1. A method of matching data, which is performed by an apparatus for matching data, the method comprising:
synchronizing acquisition time points of cone beam computed tomography (CBCT) data and 3D scan data;
conforming coordinate systems of the CBCT data and the 3D scan data;
constructing a 3D scan image in which a motion is corrected by matching depth maps created each time the 3D scan data are acquired; and
constructing a CBCT 3D image in which the motion is corrected from the CBCT data by applying motion information acquired by matching the depth maps.

2. The method of claim 1, wherein the synchronizing of the acquisition time points comprises:
conforming a frame rate of a CBCT imaging apparatus for acquiring the CBCT data and a frame rate of a 3D scan imaging apparatus for acquiring the 3D scan data; and
conforming a time point at which the CBCT imaging apparatus begins to detect the CBCT data and a time point at which the 3D scan imaging apparatus begins to capture the 3D scan data.

3. The method of claim 1, wherein the conforming of the coordinate systems comprises:
calculating a transform by using an iterative closest point (ICP) algorithm to minimize a matching error of the CBCT data and the 3D scan data;
applying the transform to the 3D scan data; and
moving the 3D scan data, to which the transform is applied, to the CBCT data.

4. The method of claim 1, wherein the constructing of the 3D scan image comprises:
creating the depth map for each frame for which the 3D scan data are acquired;
matching the two depth maps with a difference by using an ICP algorithm when it is identified that there is the difference between the created depth maps; and
setting a transform, which is calculated by the ICP algorithm, to motion information to match the two depth maps.

5. The method of claim 4, wherein the acquiring of the depth map comprises:
detecting a corresponding point between cameras or between the camera and a projector from a particular pattern when the camera of a 3D scan imaging apparatus captures a particular pattern outputted to an image capturing target from the projector of the 3D scan imaging apparatus for acquiring the 3D scan data;
acquiring depth information for each pixel of the camera with respect to the corresponding point; and
creating the depth map on the basis of the depth information.

6. The method of claim 4, wherein the constructing of the CBCT 3D image comprises:
searching for CBCT data acquired at the acquisition time point of the 3D scan data to which the motion information is set; and
performing back-projection by applying a transform, which is the motion information, to the searched CBCT data.

7. An apparatus for matching data, the apparatus comprising:
a time point synchronization unit configured to synchronize acquisition time points of cone beam computed tomography (CBCT) data and 3D scan data;
a coordinate synchronization unit configured to conform coordinate systems of the CBCT data and the 3D scan data;
a 3D scan image construction unit configured to construct a 3D scan image in which a motion is corrected by matching depth maps created each time the 3D scan data are acquired; and
a CBCT 3D image construction unit configured to construct a CBCT 3D image in which the motion is corrected from the CBCT data by applying motion information acquired by matching the depth maps.

8. A computer program stored in a computer-readable medium and configured to be executed to perform the method according to claim 1 when an instruction of the computer program is executed.
